# EUROPÄISCHE PATENTSCHRIFT

(11) **EP 1 262 170 B1**
(45) Veröffentlichungstag und Bekanntmachung des Hinweises auf die Patenterteilung: **10.05.2006**
(21) Anmeldenummer: 02011418.7
(22) Anmeldetag: 24.05.2002
(51) Int. Cl.: A61K 8/04, A61K 8/34, A61K 8/41, C07C 211/63, C11D 1/62, C11D 3/20

(54) **Zusammensetzungen, enthaltend quaternäre Ammoniumverbindungen**
Compositions comprising quaternary ammonium compounds
Compositions comprenant des ammonium quaternaires

(30) Priorität: 29.05.2001 DE 10126252
(43) Veröffentlichungstag der Anmeldung: 04.12.2002
(73) Patentinhaber: Clariant Produkte (Deutschland) GmbH, 65929 Frankfurt am Main (DE)
(72) Erfinder: Milbradt, Robert, Dr., 65189 Wiesbaden (DE); Klein, Sonja, 65795 Hattersheim (DE); Scherl, Franz Xaver, Dr., 84508 Burgkirchen (DE); Gatter, Erich, Dr., 84556 Kastl (DE); Oberhauser, Adelgunde, 84524 Neuötting (DE)

(56) Entgegenhaltungen:
- EP-A- 0 655 236
- WO-A-00/28950
- WO-A-91/12880
- US-A- 5 102 655
- US-A- 5 888 488
- US-A- 5 929 263
- DATABASE WPI Section Ch, Week 199932 Derwent Publications Ltd., London, GB; Class D21, AN 1994-354596 XP002213719 -& JP 06 279233 A (HOYU KK), 5. Juli 1999 (1999-07-05)

## Beschreibung

Die Erfindung betrifft Zusammensetzungen, enthaltend quaternäre Ammoniumverbindungen, die einen niedrigen Stockpunkt, gute Löslichkeit bzw. Dispergierbarkeit in wässrigen Medien und einen niedrigen Flammpunkt besitzen und somit hervorragend zur Konfektionierung von quaternären Ammoniumverbindungen geeignet sind.

Kosmetische Mittel, wie z.B. Haarbehandlungsmittel, enthalten häufig in Wasser schwerlösliche quaternäre Ammoniumverbindungen, die eine langkettige Alkyl- oder Alkenylgruppe aufweisen. Solche Mittel sind üblicherweise als wässrige Dispersionen, Emulsionen, Mikroemulsionen, Gele oder auch in Aerosolform konfektioniert und werden z.B. als Shampoos, Haarkuren, Haarspülungen etc. eingesetzt.

Für den Hersteller solcher Mittel ist es von großem Vorteil, die quaternären Ammoniumverbindungen als Compounds oder Formulierungen in Form von Flakes, Pellets oder Pasten bereitzustellen, die bei hohem kationischen Wirkstoffanteil einen niedrigen Stockpunkt sowie gute Löslichkeit bzw. Dispergierbarkeit in wässrigen Medien besitzen.
Gemäß dem Stand der Technik können die obigen Anforderungen durch die Zugabe von kurzkettigen Alkoholen, insbesondere von Isopropanol in Mengen von 15 bis 20 Gew.-%, erreicht werden. Aufgrund ihrer niedrigen Siede- und Flammpunkte sind solche kurzkettigen Alkohole jedoch problematisch.
Wie in WO 00/28950 beschrieben, können die kurzkettigen Alkohole durch lineare Fettalkohole (z.B. Cetyl-, Lauryl-, Behenyl- oder Stearylalkohol) ersetzt werden. Um den Stockpunkt bzw. Schmelzpunkt der Mischungen auf Temperaturen unterhalb 100°C herabzusetzen werden zusätzlich Glykole, wie z.B. Propylenglykol oder 1,3-Butandiol, zugesetzt. In der WO 00/28950 wird weiterhin hervorgehoben, dass es sich bei den Fettalkoholen vorteilhafterweise um homogene Fettalkohole handelt, die weniger als ca. 10 Gew.-% eines anderen Fettalkohols enthalten.

Überraschenderweise wurde nun gefunden, dass Zusammensetzungen, enthaltend quaternäre Ammoniumverbindungen und verzweigte langkettige Alkohole, bevorzugt Mischungen von verzweigten und unverzweigten langkettigen Alkoholen, und niedermolekulare mehrwertige Alkohole, niedrige Stock- und Schmelzpunkte, gute Löslichkeit bzw. Dispergierbarkeit in wässrigen Medien und einen niedrigen Flammpunkt besitzen. Derartige Zusammensetzungen eignen sich damit hervorragend zur Konfektionierung von quaternären Ammoniumverbindungen.

Gegenstand der Erfindung sind Zusammensetzungen, enthaltend
a) 30 bis 90 Gew.-% mindestens einer quaternären Ammoniumverbindung gemäß Formel (1) 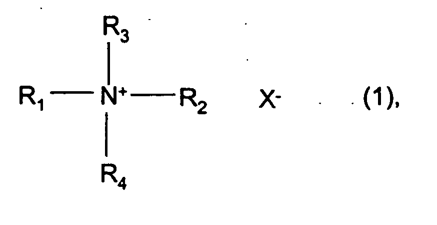 wobei
   R₁ für eine unverzweigte oder verzweigte Alkyl- oder Alkenylgruppe mit 12 bis 36 Kohlenstoffatomen, eine Gruppe R₅CONH(CH₂)ₙ- oder eine Gruppe R₅COO(CH₂)ₙ₋steht, wobei R₅ für eine Alkyl- oder Alkenylgruppe mit 12 bis 36 Kohlenstoffatomen und n für eine Zahl von 1 bis 8 stehen,
   und
   R₂, R₃ und R₄ unabhängig voneinander gleich oder verschieden sein können und für eine -CH₃, CH₃CH₂-,CH₃CH₂CH₂-, CH₃CH₂CH₂CH₂-, CH₂CH₂OH oder
   -CH₂CH(OH)CH₂OH-Gruppe stehen
   und
   X⁻ für ein Anion steht,
   und
b) mindestens einen verzweigten Alkohol b1) mit 8 bis 36 Kohlenstoffatomen oder eine Mischung aus mindestens einem verzweigten Alkohol b1) und mindestens einem unverzweigten Alkohol b2) mit 8 bis 36 Kohlenstoffatomen,
wobei sie frei sind von mehr wertigen Alkoholen mit 2 bis 6 Kohlenstoffatomen und in Form von Pellets oder Flakes vorliegen.
Die verzweigten Alkohole b1) und unverzweigten Alkohole b2) können gesättigt oder ungesättigt sein.

Besonders vorteilhafte anwendungstechnische Eigenschaften zeigen Zusammensetzungen, die als Komponente b) eine Mischung aus mindestens einem verzweigten Alkohol b1) und mindestens einem unverzweigten Alkohol b2) enthalten.

Der Anteil an quaternären Ammoniumverbindungen a), bezogen auf die fertigen Zusammensetzungen, beträgt bevorzugt 40 bis 90 Gew.-%, besonders bevorzugt 40 bis 80 Gew.-%, insbesondere bevorzugt 45 bis 75 Gew.-%, ganz besonders bevorzugt 45 bis 65 Gew.-%.
Überraschenderweise zeigte sich, dass die Zusammensetzungen vorteilhafterweise hohe Gewichtsanteile an quaternären Ammoniumverbindungen a) bei gleichzeitig niedrigen Schmelz- bzw. Stockpunkten enthalten können.

Als quaternäre Ammoniumverbindungen a) bevorzugt sind (C₁₂-C₃₆)-Alkyltrimethylammoniumverbindungen, besonders bevorzugt (C₁₄-C₃₀)-Alkyltrimethylammoniumverbindungen, insbesondere bevorzugt (C₁₆-C₂₄)-Alkyltrimethylammoniumverbindungen.
Insbesondere bevorzugt sind Alkyltrimethylammoniumverbindungen bei denen der Alkylrest einen Behenyl-, Erucyl-, Cetyl- oder Stearylrest darstellt.
Beim Anion X⁻ in Formel (1) kann es sich um ein beliebiges ladungsausgleichendes Anion handeln; bevorzugt sind Chlorid, lodid, Bromid, Methosulfat, Hydrogensulfat, Lactat und/oder Citrat, besonders bevorzugt Chlorid und Methosulfat.
Ganz besonders geeignet als quaternäre Ammoniumverbindung a) ist Behenyltrimethylammoniumchlorid.

Der Anteil der Alkoholkomponente b) beträgt, bezogen auf die fertigen Zusammensetzungen, bevorzugt 10 bis 70 Gew.-%, besonders bevorzugt 20 bis 60 Gew.-%, insbesondere bevorzugt 25 bis 50 Gew.-%.

Enthalten die Zusammensetzungen verzweigte Alkohole b1) und unverzweigte Alkohole b2), dann beträgt das Gewichtsverhältnis von verzweigten Alkoholen b1) zu unverzweigten Alkoholen b2) bevorzugt 90 : 10 bis 10 : 90, besonders bevorzugt 70 : 30 bis 30 : 70, insbesondere bevorzugt 60 : 40 bis 40 : 60.
Bevorzugt besitzen die verzweigten Alkohole b1) und die unverzweigten Alkohole b2) 10 bis 24, besonders bevorzugt 12 bis 18, insbesondere bevorzugt 14 bis 16 Kohlenstoffatome. Die verzweigten Alkohole b1) sind bevorzugt einfach verzweigt.

Als verzweigte Alkohole b1) besonders geeignet sind Guerbetalkohole, 2-Methyl-1-decanol, 2-Ethyl-1-nonanol, 2-Propyl-1-octanol, 2-Butyloctanol, 2-Butyl-decanol, 2-Hexyloctanol, 2-Hexyldecanol, 2-Octyldecanol, 2-Hexyldodecanol, 2-Octyldodecanol, 2-Decyltetradecanol, 2-Butyl-1-heptanol, 2-Dodecylhexadecanol, Tetradecyloctadecanol, 2-Tetradecyleicosanol, 2-Hexadecyloctadecanol, 2-Hexadecyleicosanol, 2-Methyltridecanol, 2-Ethyldodecanol, 2-Propylundecanol, 2-Pentylnonanol, 2-Heptylheptanol, 2-Methyltetradecanol, 2-Ethyltridecanol, 2-Propyldodecanol, 2-Butylundecanol, 2-Pentyldecanol, 2-Hexylnonanol, 2-Heptyloctanol, Isotridecylalkohol und Mischungen derselben.

Besonders bevorzugt sind Zusammensetzungen, die als verzweigte Alkohole b1) Alkoholgemische entsprechend den kommerziell erhältlichen ® Isalchem-Typen (Fa. Sasol) enthalten.

Bevorzugt als verzweigte Alkohole b1) für den gemeinsamen Einsatz mit unverzweigten Alkoholen b2 sind 2-Methyltridecanol, 2-Ethyldodecanol, 2-Propylundecanol, 2-Butyldecanol, 2-Pentylnonanol, 2-Hexyloctanol, 2-Heptylheptanol, 2-Methyltetradecanol, 2-Ethyltridecanol, 2-Propyldodecanol, 2-Butylundecanol, 2-Pentyldecanol, 2-Hexylnonanol und 2-Heptyloctanol.

Bevorzugt als unverzweigte Alkohole b2) sind Fettalkohole, besonders bevorzugt Lauryl-, Myristyl-, Cetyl-, Stearyl, Eicosanyl- und Behenylalkohol. Ebenfalls bevorzugt sind Nonanol, Undecanol, Tridecanol, Pentadecanol und Heptadecanol.

In einer besonders bevorzugten Ausführungsform enthalten die Zusammensetzungen verzweigte Alkohole b1) mit 14 bis 16 Kohlenstoffatomen und unverzweigte Alkohole b2) mit 14 bis 16 Kohlenstoffatomen, wobei das Gewichtsverhältnis, bezogen auf die fertigen Zusammensetzungen, von verzweigten Alkoholen b1) zu unverzweigten Alkoholen b2) 70 : 30 bis 30 : 70, besonders bevorzugt 65 : 35 bis 55 : 45, insbesondere bevorzugt 60 : 40, beträgt und das Gewichtsverhältnis von Alkoholen mit 14 Kohlenstoffatomen zu Alkoholen mit 15 Kohlenstoffatomen im Bereich von 55 : 45 bis 65 : 35 liegt.

Besonders bevorzugt sind Zusammensetzungen, die als Mischungen von verzweigten Alkoholen b1) und unverzweigten Alkoholen b2) Alkoholgemische entsprechend den ® LIAL-Typen (z.B. ® LIAL 145 und 123, Fa. Sasol) enthalten. Die Alkohole aus den ® LIAL-Typen werden typischerweise nach dem Oxo-Prozess hergestellt.

Überraschenderweise wurde gefunden, daß bei Einsatz der Alkohole b1) die Zusammensetzungen frei sein können von mehrwertigen Alkoholen mit 2 bis 6 Kohlenstoffatomen, wie z.B. Glykolen (z.B. Ethylenglykol und Propylenglykol), Butandiolen (z.B. 1,3-Butandiol), Glycerin, Thioglycerin, Sorbitol, Xylitol und Mannitol.

Optional können die erfindungsgemäßen Zusammensetzungen zur Verbesserung der anwendungstechnischen Eigenschaften unverzweigte oder verzweigte Monoalkohole mit 1 bis 4 Kohlenstoffatomen enthalten. Bevorzugt als Monoalkohole sind Ethanol, Propanol, Isopropanol, Butanol, Isobutanol und t-Butanol, besonders bevorzugt Isopropanol. Bevorzugt enthalten die Zusammensetzungen, bezogen auf die fertigen Zusammensetzungen, weniger als 5 Gew.-%, besonders bevorzugt weniger als 1 Gew.-%, an derartigen Monoalkoholen.
In einer bevorzugten Ausführungsform sind die Zusammensetzungen frei von unverzweigten oder verzweigten Monoalkoholen mit 1 bis 4 Kohlenstoffatomen.

Die erfindungsgemäßen Zusammensetzungen besitzen bevorzugt Stockpunkte unterhalb 100 °C, besonders bevorzugt unterhalb 95°C, insbesondere bevorzugt unterhalb 90°C, ganz besonders bevorzugt unterhalb 85°C.

Der Flammpunkt der erfindungsgemäßen Zusammensetzungen liegt bevorzugt oberhalb 80°C, besonders bevorzugt oberhalb 100°C.

Bei den erfindungsgemäßen Zusammensetzungen handeltes sich um Pellets und Flakes.

In einer bevorzugten Ausführungsform werden die erfindungsgemäßen Zusammensetzungen dadurch hergestellt, dass eine Mischung hergestellt wird, enthaltend
i) mindestens eine quaternäre Ammoniumverbindung a), gegebenenfalls enthaltend einen verzweigten oder unverzweigten Monoalkohol mit 1 bis 4 Kohlenstoffatomen,
ii) mindestens einen verzweigten Alkohol b1) mit 8 bis 36 Kohlenstoffatomen oder eine Mischung aus mindestens einem verzweigten Alkohol b1) und mindestens einem unverzweigten Alkohol b2) mit 8 bis 36 Kohlenstoffatomen und
iii) gegebenenfalls mindestens einen unverzweigten oder verzweigten Monoalkohol mit 1 bis 4 Kohlenstoffatomen.

In einer bevorzugten Ausführungsform werden die Komponenten i) bis iii) vermischt und anschließend, gegebenenfalls unter Rühren, erwärmt. Dabei wird die Temperatur so gewählt, dass eine Schmelze vorliegt. Bevorzugt sind Temperaturen von 70 bis 120 °C, besonders bevorzugt 80 bis 110°C. In einer anderen bevorzugten Ausführungsform wird die Komponente i) als Schmelze vorgelegt.

Die quaternären Ammoniumverbindungen a) der Komponente i) können in bekannter Weise durch Alkylierung eines tertiären Amins in Gegenwart mindestens eines unverzweigten oder verzweigten Monoalkohols mit 1 bis 4 Kohlenstoffatomen, bevorzugt Ethanol, Propanol, Isopropanol, Butanol, Isobutanol und t-Butanol, besonders bevorzugt Isopropanol, hergestellt werden. Die quaternären Ammoniumverbindungen werden bevorzugt als Pellets oder besonders bevorzugt als Pulver eingesetzt. In einer bevorzugten Ausführungsform enthalten die quaternären Ammoniumverbindungen weniger als 5 Gew.-%, bevorzugt weniger als 2 Gew.-%, an Monoalkoholen. In einer weiteren bevorzugten Ausführungsform enthalten die quaternären Ammoniumverbindungen 10 bis 25 Gew.-% an Monoalkoholen. Die erfindungsgemäßen Zusammensetzungen besitzen bevorzugt einen Gesamtgehalt an unverzweigten oder verzweigten Monoalkoholen mit 1 bis 4 Kohlenstoffatomen von weniger als 5 Gew.-%, besonders bevorzugt weniger als 1 Gew.-%.
In einer ebenfalls bevorzugten Ausführungsform sind die erfindungsgemäßen Zusammensetzungen frei von unverzweigten oder verzweigten Monoalkoholen mit 1 bis 4 Kohlenstoffatomen.
Zur Einstellung des gewünschten Gehalts an Monoalkoholen in den erfindungsgemäßen Zusammensetzungen werden die Komponenten i) und/oder iii) entsprechend gewählt bzw. bemessen und/oder man entfernt die Monoalkohole teilweise oder ganz vorab aus der Komponente i).
In einer weiteren Ausführungsform werden die Monoalkohole nachträglich aus den erfindungsgemäßen Zusammensetzungen bis auf den gewünschten Restgehalt entfernt. Bevorzugt werden die Monoalkohole bei 700 bis 10 mbar, bevorzugt 400 bis 70 mbar, und 60 bis 90°C abgezogen.
Ebenso können die Monoalkohole bei Normaldruck in geeigneten Verdampfungseinrichtungen (z.B. Dünnschicht-Verdampfer) bei Temperaturen bis 120 °C abdestilliert werden.

Überraschenderweise wurde gefunden, dass die erfindungsgemäßen Zusammensetzungen auch "in situ" durch Alkylierung von
i) mindestens einem tertiären Amin NR₁R₂R₃,
   wobei R₁ für eine unverzweigte oder verzweigte Alkyl- oder Alkenylgruppe mit 12 bis 36 Kohlenstoffatomen, eine Gruppe R₅CONH(CH₂)ₙ- oder eine Gruppe R₅COO(CH₂)ₙ-steht, wobei R₅ für eine Alkyl- oder Alkenylgruppe mit 12 bis 36 Kohlenstoffatomen und n für eine Zahl von 1 bis 8 stehen,
   und R₂ und R₃ unabhängig voneinander gleich oder verschieden sein können und für CH₃-, CH₃CH₂-, CH₃CH₂CH₂-, CH₃CH₂CH₂CH₂- oder
   -CH₂CH₂(OH) stehen, durch
ii) mindestens ein Alkylierungsmittel, ausgewählt aus
   a) R₄X, wobei R₄ für -CH₃, CH₃CH₂-, CH₃CH₂CH₂-, CH₃CH₂CH₂CH₂- oder -CH₂CH(OH)CH₂(OH) steht und X für Cl, I, Br, OSO₃H oder Methosulfat steht, und/oder
   b) Ethylenoxid und einer Säure HX, wobei X für Cl, I, Br, OSO₃H, Citrat oder Lactat steht,
   in Gegenwart von
iii) mindestens einem verzweigten Alkohol b1) mit 8 bis 36 Kohlenstoffatomen oder einer Mischung aus mindestens einem verzweigten Alkohol b1) und einem unverzweigten Alkohol b2) mit 8 bis 36 Kohlenstoffatomen und
iv) gegebenenfalls mindestens einem unverzweigten oder verzweigten Monoalkohol mit 1 bis 4 Kohlenstoffatomen
hergestellt werden können.

Bevorzugt werden bei der Umsetzung die Einsatzmengen an verzweigten Alkoholen b1), unverzweigten Alkoholen b2) und gegebenenfalls unverzweigten oder verzweigten Monoalkoholen mit 1 bis 4 Kohlenstoffatomen entsprechend der schon vorab beschriebenen bevorzugten Zusammensetzung der erfindungsgemäßen Zusammensetzungen gewählt. Die Gehalte können auch durch nachträgliche Zugabe oder Entfernen eingestellt werden.

In einer bevorzugten Ausführungsform werden bei der Umsetzung keine unverzweigten oder verzweigten Monoalkohole mit 1 bis 4 Kohlenstoffatomen eingesetzt.

Die nach den oben beschriebenen Verfahren hergestellten erfindungsgemäßen Zusammensetzungen werden als homogene oder inhomogene Schmelze durch Abkühlen in Pellets, Flakes, überführt.

Die erfindungsgemäßen Zusammensetzungen eignen sich allgemein zur Herstellung von Mitteln, enthaltend quaternäre Ammoniumverbindungen.
Besonders geeignet sind die Zusammensetzungen zur Herstellung von kosmetischen, dermatologischen und pharmazeutischen Mitteln.

Insbesondere eignen sie sich für die Herstellung von Haarbehandlungsmitteln. Gegenstand der Erfindung ist demnach auch die Verwendung der erfindungsgemäßen Zusammensetzungen zur Herstellung von Mitteln, bevorzugt kosmetischen, dermatologischen und pharmazeutischen Mitteln, insbesondere Haarbehandlungsmitteln, enthaltend quaternäre Ammoniumverbindungen. Beispiele für bevorzugte Mittel sind Shampoos, rinse-off-Haarconditionierer, Cremespülungen, Klarspülungen, Haarkuren, Haarfärbe- und Haartönungsmittel, Dauerwellmittel, Haargele, Haarconditionierer in Aerosol-, Spray- und Fluidform, 2-in-1 Duschbäder, Cremeduschbäder, Hautpflegemittel, Tagescremes, Nachtcremes, Pflegecremes, Nährcremes, Bodylotions und Salben.

Die kosmetischen, dermatologischen und pharmazeutischen Mittel enthalten die erfindungsgemäßen Zusammensetzungen, bezogen auf die fertigen Mittel, bevorzugt in Mengen von 0,1 bis 15 Gew.-%, besonders bevorzugt 1 bis 10 Gew.-%, insbeondere bevorzugt 1 bis 7 Gew.-%.

Die kosmetischen, dermatologischen und pharmazeutischen Mittel können als weitere Hilfs- und Zusatzstoffe alle üblichen Tenside, Ölkörper, Emulgatoren und Co-Emulgatoren, kationischen Polymere, Filmbildner, Überfettungsmittel, feuchtigkeitsspendende Mittel, Stabilisatoren, biogene Wirkstoffe, Konservierungsmittel, Perlglanzmittel, Farb- und Duftstoffe, Lösungsmittel, Glycerin, Hydrotrope, Trübungsmittel, Verdickungsmittel, Dispergiermittel, Eiweißderivate, wie z.B. Gelatine, Collagenhydrolysate, Polypeptide auf natürlicher und synthetischer Basis, Eigelb, Lecithin, Lanolin und Lanolinderivate, Silicone, deodorierende Mittel, Stoffe mit keratolytischer und keratoplastischer Wirkung, Enzyme, Trägersubstanzen, Antioxidantien, UV-Lichtschutzfilter, Pigmente und Metalloxide, sowie antimikrobiell wirkende Agentien enthalten.

Als Tenside können anionische, kationische, nichtionische, amphotere und/oder zwitterionische Tenside verwendet werden. Bevorzugte nichtionische Tenside enthalten als hydrophile Gruppe eine Polyolgruppe, eine Polyolalkenylethergruppe oder eine Kombination aus Polyol- und Polyglykolethergruppen. Bevorzugt sind Anlagerungsprodukte aus von 2 bis 30 Mol Ethylenoxid, 2 bis 30 Mol Ethylenoxid zusammen mit bis 5 Mol Propylenoxid oder von bis 5 Mol Propylenoxid an lineare Fettalkohole mit 8 bis 22 C-Atomen und Alkylphenolen mit 8 bis 15 C-Atomen in der Alkylgruppe, (C₁₂-C₁₉)-Fettsäuremono- und diester von Anlagerungsprodukten von 1 bis 30 Mol Ethylenoxid an Glycerin, Glycerinmono- und -diester und Sorbitanmono- und -diester von gesättigten und ungesättigten (C₈-C₁₈)-Fettsäuren und deren Ethylenoxidanlagerungsprodukte, (C₈-C₁₈)-Alkylmono- und - oligoglykoside und deren ethoxylierte Analoga, Anlagerungsprodukte von 10 bis 60 Mol Ethylenoxid an Ricinusöl und gehärtetes Ricinusöl, ethoxylierte und nicht ethoxylierte Mono-, Di- und Trialkylmonophosphor-säureester, insbesondere Mono-, Di- und Tri-(Lauryltetraglycolether)-o-Phosphor-säureester und Mono-, Di- und Tri-(Cetyltetraglycolether)-o-Phosphorsäureester.

Bevorzugte amphotere Tenside tragen eine (C₈-C₁₈)-Alkyl- oder -Acylgruppe und mindestens eine freie Aminogruppe und mindestens eine -COOH- oder -SO₃H-Gruppe. Bevorzugt sind N-Acylglycine, N-Alkylpropionsäure, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils 8 bis 18 C-Atomen in der Alkylgruppe. Besonders bevorzugte sind N-Kokosalkylaminopropionat, Kokosacylaminoethylaminopropionat und (C₁₂-C₁₈)-Alkylsarcosine.

Besonders geeignete zwitterionische Tenside sind Betaine, wie beispielsweise die N-Alkyl-N,N-dimethylammoniumglycinate, z.B. Kokosalkyldimethylammoniumglycinate, N-Acyl-aminopropyl-N-N-dimethylammoniumglycinate, z.B. Kokosacylaminopropyl-dimethylammoniumglycinat, 2-Alkyl-3-carboxymethyl-3-hydroxyethylimidazoline mit jeweils 8 bis 18 C-Atomen in der Alkyl- oder Acylgruppe und das Kokosacylamino-ethylhydroxyethylcarboxymethylglycinat.

Bevorzugt enthalten die Mittel Tensid-Mischungen, besonders bevorzugt sind Mischungen aus nichtionischen und zwitterionischen oder amphoteren Tensiden in einem Gewichtsverhältnis von 5 zu 1 bis 1 zu 5 oder Mischungen aus nichtionogenen Tensiden und beliebigen Mischungen aus zwitterionischen und amphoteren Tensiden in einem Gewichtsverhältnis von 5 zu 1 bis 1 zu 5.

Als Ölkörper eignen sich alle bekannten Öle, Fette und Wachse mineralischer, tierischer, pflanzlicher und synthetischer Herkunft. Bevorzugt sind als Öl- und Fettkomponenten Diallylether mit insgesamt 12 bis 24 C-Atomen, Fettsäureester mit insgesamt 12 bis 26 C-Atomen, flüssige Kohlenwasserstoffe mit 10 bis 32 C-Atomen und Gemische davon. Geeignete Fettsäureester sind z.B. Methylpalmitat, Ethyloleat, Isopropylmyristat, n-Hexyllaurat, n-Butylstearat und Cetyl-/ Stearylisononanoat. Besonders bevorzugt sind Paraffinöle, Vaseline, Pflanzenöle, synthetische Triglyceride wie z.B. Glyceryltricaprylat, sowie Silikonöle.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden. Als Konsistenzgeber kommen Fettalkohole mit 12 bis 22, vorzugsweise 12 bis 18 Kohlenstoffatomen sowie Partialglyceride in Betracht.

Als weitere Verdickungsmittel können Polysaccharide, insbesondere Xantham-Gum, Guar-Guar, Agar-Agar, Alginate, Carboxymethylcellulose, Hydroxyethylcellulose, höhermolekulare Polyethylenglykolmono- und -diester von Fettsäuren, Polyacrylate, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylpropan, Fettalkoholethoxylate oder Alkyloligoglucoside, sowie Elektrolyte wie Kochsalz und Ammoniumchlorid eingesetzt werden.

Geeignete Siliconverbindungen sind beispielsweise Dimethylpolysiloxan, Methylphenylpolysiloxane, cyclische Silicone sowie amino-, fettsäure-, alkohol-, polyether-, epoxy-, fluor- und/ oder alkylmodifizierte Siliconverbindungen, die bei Raumtemperatur sowohl flüssig als auch harzförmig vorliegen können.

Unter biogenen Wirkstoffen sind beispielsweise Bisabolol® , Allantoin® , Phytantriol® , Panthenol® , AHA-Säuren, Pflanzenextrakte und Vitaminkomplexe zu verstehen.

Als Antischuppenmittel können Climbazol® , Octopirox® , Oxiconazol® und Zinkpyrethion® eingesetzt werden.

Gebräuchliche Filmbildner sind Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Zur Verbesserung des Fließverhaltens können ferner Hydrotrope wie beispielsweise Ethanol, Isopropylalkohol, Propylenglykol oder Glucose eingesetzt werden.

Als Konservierungsmittel eignen sich beispielsweise Phenoxyethanol, Formaldehydlösung, Parabene, Pentandiol und Sorbinsäure.

Als feuchtigkeitsspendende Substanz stehen beispielsweise Isopropylpalmitat, Glycerin und/oder Sorbitol zu Verfügung.

Der Gesamtanteil an Hilfs- und Zusatzstoffen in den Mitteln beträgt bevorzugt 1 bis 50 Gew.-%, besonders bevorzugt 5 bis 40 Gew.-%.

Die nachfolgenden Beispiele dienen der Erläuterung der Erfindung, ohne sie jedoch einzuschränken. Der Quat-Aktivgehalt der erfindungsgemäßen Zusammensetzungen wurden durch Kation-Titration bestimmt. Die Stockpunkte wurde durch langsames Absenken der Temperatur ermittelt.

### Beispiel 1:

120,34 g ® Genamin KDMP (Behenyltrimethylammoniumchlorid Pellets mit einem Gehalt von ca. 19 % Isopropanol, Fa. Clariant GmbH) wurden in 100,11 g ® LIAL 145 (Mischung aus einfach verzweigten und linearen Fettalkoholen mit einer Alkylkettenlänge von 14 bis 15 C-Atomen; Fa. Sasol) in einem 1000 ml-Rundkolben gemischt und in einem Ölbad bei 70- 90°C aufgeschmolzen. Nachdem sich eine homogene Schmelze gebildet hatte wurde am Rotationsverdampfer ein Vakuum von zunächst ca. 700 mbar angelegt und das Isopropanol während etwa 3 Stunden abdestilliert. Dabei wurde das Vakuum kontinuierlich auf etwa 22 mbar erhöht. Anschließend entfernte man Reste des flüchtigen Lösemittels während 2 Stunden bei 90°C Badtemperatur und 16 mbar Vakuum. Man erhielt eine klare Lösung, die bei 80-84°C erstarrte und bei 90 °C wieder vollständig geschmolzen vorlag. Bei 25°C war die Zusammensetzung wachsartig fest. Der Quat-Aktivgehalt betrug 47 Gew.-%.

### Beispiel 2:

Analog Beispiel 1 wurde eine Zusammensetzung aus
57 Gew.-% Behenyltrimethylammoniumchlorid, 40 Gew.-% ® LIAL 145 und
3 Gew.-% Isopropanol hergestellt. Man erhielt eine klare Lösung, die bei 80-84°C erstarrte und bei 90 °C wieder vollständig geschmolzen vorlag. Bei 25 °C war die Zusammensetzung wachsartig fest. Der Quat-Aktivgehalt betrug 57 Gew.-%.

### Beispiel 3:

Analog Beispiel 1 wurde eine Zusammensetzung aus
64 Gew.-% Behenyltrimethylammoniumchlorid, 33 Gew.-% ® LIAL 145 und
3 Gew.-% Isopropanol hergestellt. Man erhielt eine klare Lösung, die bei 80-84°C erstarrte und bei 90 °C wieder vollständig geschmolzen vorlag. Bei 25 °C war die Zusammensetzung wachsartig fest. Der Quat-Aktivgehalt betrug 64 Gew.-%.

### Beispiel 4:

® Genamin KDMP (Behenyltrimethylammoniumchlorid Pellets mit einem Gehalt von ca. 19 % Isopropanol, Fa. Clariant GmbH) wurde in einem Vakuumofen zur Entfernung des Lösemittels 14 Stunden bei einer Temperatur 74°C und ca. 200 mbar Druck bis zur Gewichtskonstanz getrocknet. (Der Gewichtsverlust entsprach dabei der erwarteten Lösemittelmenge von ca. 19 %). Die so getrockneten Pellets wurden zu einem Pulver gemahlen und gesiebt (Siebweite: 630 µm). 4,9 g getrocknetes und gesiebtes Behenyltrimethylammoniumchlorid wurde in einer 50 ml- Pulverflasche aus Glas vorgelegt und mit 5,1 g ® LIAL 145 (Fa. Sasol) versetzt. Nach Verschließen der Flasche wurde die Mischung 12 Stunden auf ca. 100°C erhitzt. Während dieser Zeit wurde die Probe mit einem Spatel mehrmals gerührt, um die Mischung zu homogenisieren. Man erhielt eine klare, leicht gelbliche Lösung, die bei 79-80°C erstarrte und bei 85 °C wieder vollständig geschmolzen vorlag. Der Quat-Aktivgehalt betrug 49 Gew.-%.

### Beispiel 5:

Analog Beispiel 4 wurde eine Zusammensetzung aus
60 Gew.-% Behenyltrimethylammoniumchlorid und 40 Gew.-% ® LIAL 145 hergestellt. Man erhielt eine leicht trübe Schmelze, die bei 80-82°C erstarrte und bei 90°C wieder vollständig geschmolzen vorlag. Bei 25°C war die Zusammensetzung wachsartig fest. Der Quat-Aktivgehalt betrug 60 Gew.-%.

### Vergleichsbeispiel 4a):

Eine Zusammensetzung aus 49 Gew.-% Behenyltrimethylammoniumchlorid und 51 Gew.-% Cetearylalkohol besaß einen Schmelzpunkt von 105-108°C (s.h. WO 00/28950). Der Quat-Aktivgehalt betrug 49 Gew.-%.

### Formulierungsbeispiele für kosmetische Formulierungen:

Die erfindungsgemäße Zusammensetzung aus Beispiel 4 wurde aufgeschmolzen und durch Auftropfen auf eine kalte Metallplatte pelletiert. Die so erhaltenen Pellets wurden dann in die entsprechenden Formulierungen eingearbeitet.

### Beispiel 6: Cremespülung

| | | |
|---|---|---|
| A | Zusammensetzung aus Bsp. 4 | 3 Gew.-% |
| | ® HOSTAPHAT KL 340 D | 1,5 Gew.-% |
| | (Trilaureth-4 Phosphate, Fa. Clariant) | |
| | Cetylalkohol | 3 Gew.-% |
| | Paraffinöl | 1 Gew.-% |
| B | Wasser | 91,5 Gew.-% |
| C | Citronensäure | q.s. |

### Herstellung:

I) A bei 75°C aufschmelzen
II) B auf 75°C erwärmen
III) B in A einrühren und Abkühlen
IV) pH-Wert mit C auf pH = 4 einstellen

### Beispiel 7: O/W-Handcreme

| | | |
|---|---|---|
| A | Zusammensetzung aus Bsp. 4 | 4 Gew.-% |
| | ® HOSTACERIN DGSB | 6 Gew.-% |
| | (PEG-4 Polyglyceryl-2 Stearate, Fa. Clariant) | |
| | Paraffinöl, hochviskos | 10 Gew.-% |
| | Isopropylpalmitat | 10 Gew.-% |
| B | Wasser | 69,6 Gew.-% |
| C | Konservierungsmittel Parfüm | q.s. 0,4 Gew.-% |

### Herstellung:

I) A bei 80°C aufschmelzen
II) B auf 80°C erwärmen
III) B in A einrühren und Abkühlen
IV) C bei 35°C zugeben

### Beispiel 8: Haarconditionierer mit Perlglanzeffekt

| | | |
|---|---|---|
| A | Zusamensetzung aus Bsp. 4 | 3 Gew.-% |
| | ® Genamin KSL | 9 Gew.-% |
| | (PEG- 5 Stearyl Ammonium Lactate, Fa. Clariant) | |
| | ® Hostaphat KL 340 D | 1,5 Gew.-% |
| | (Trilaureth- 4 Phosphate, Fa. Clariant) | |
| | Jojobaöl | 1,0 Gew.-% |
| B | ® Tylose H 100 000 YP2 | 1,5 Gew.-% |
| | (Hydroxyethylcellulose, Fa. Clariant | |
| C | Wasser | ad 100 % |
| D | Parfüm | 0,50 % |
| | Panthenol | 0,50 % |
| | ® Genapol PDC | 4 Gew.-% |
| | (Glycol Distearate, Laureth- 4, Cocamidopropyl Betaine, Glimmer und Titandioxid, Fa. Clariant) | |
| E | Citronensäure | q.s. |

### Herstellung:

I) A wird auf 75°C erwärmt
II) B wird sorgfältig in C gelöst und auf etwa 75°C erwärmt
III) II wird unter Rühren zu I gegeben
IV) Unter Rühren abkühlen lassen, bei 30°C D in III geben
V) pH-Wert mit E auf pH = 4 einstellen

## Patentansprüche

1. Zusammensetzungen, enthaltend, bezogen auf die fertigen Zusammensetzungen,
a) 30 bis 90 Gew.-% mindestens einer quaternären Ammoniumverbindung gemäß Formel (1) 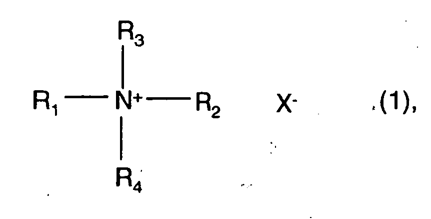 wobei
R₁ für eine unverzweigte oder verzweigte Alkyl- oder Alkenylgruppe mit 12 bis 36 Kohlenstoffatomen, eine Gruppe R₅CONH(CH₂)ₙ- oder eine Gruppe R₅COO(CH₂)ₙ₋steht, wobei R₅ für eine Alkyl- oder Alkenylgruppe mit 12 bis 36 Kohlenstoffatomen und n für eine Zahl von 1 bis 8 stehen,
und
R₂, R₃ und R₄ unabhängig voneinander gleich oder verschieden sein können und für eine -CH₃, CH₃CH₂-, CH₃CH₂CH₂-, CH₃CH₂CH₂CH₂-, CH₂CH₂OH oder
CH₂CH(OH)CH₂OH-Gruppe stehen
und
X⁻ für ein Anion steht,
und
b) mindestens einen verzweigten Alkohol b1) mit 8 bis 36 Kohlenstoffatomen oder eine Mischung aus mindestens einem verzweigten Alkohol b1) und mindestens einem unverzweigten Alkohol b2) mit 8 bis 36 Kohlenstoffatomen,
**dadurch gekennzeichnet, dass** sie frei sind von mehrwertigen Alkoholen mit 2 bis 6 Kohlenstoffatomen und in Form von Pellets oder Flakes vorliegen.

2. Zusammensetzungen nach Anspruch 1, **dadurch gekennzeichnet, dass** sie als Komponente b) eine Mischung aus mindestens einem verzweigten Alkohol b1) und mindestens einem unverzweigten Alkohol b2) enthalten.

3. Zusammensetzungen nach Anspruch 1 und/oder 2, **dadurch gekennzeichnet, dass** der Gehalt an quaternären Ammoniumverbindungen a), bezogen auf die fertigen Zusammensetzungen, 40 bis 90 Gew.-%, bevorzugt 40 bis 80 Gew.-%, besonders bevorzugt 45 bis 75 Gew.-%, beträgt.

4. Zusammensetzungen nach mindestens einem der Ansprüche 1 bis 3, **dadurch gekennzeichnet, dass** es sich bei den quaternären Ammoniumverbindungen a) um (C₁₂-C₃₆)-, bevorzugt (C₁₄-C₃₀)-, besonders bevorzugt (C₁₆-C₂₄)-Alkyltrimethylammoniumverbindungen, handelt.

5. Zusammensetzungen nach mindestens einem der Ansprüche 1 bis 4, **dadurch gekennzeichnet, dass** es sich beim Anion X⁻ in Formel (1) um Chlorid, Iodid, Bromid, Methosulfat, Hydrogensulfat, Lactat und/oder Citrat, bevorzugt Chlorid und/oder Methosulfat, handelt.

6. Zusammensetzungen nach mindestens einem der Ansprüche 1 bis 5, **dadurch gekennzeichnet, dass** es sich bei der quaternären Ammoniumverbindung a) um Behenyltrimethylammoniumchlorid handelt.

7. Zusammensetzungen nach mindestens einem der Ansprüche 1 bis 6, **dadurch gekennzeichnet, dass** der Gehalt an Alkoholen b), bezogen auf die fertigen Zusammensetzungen, 10 bis 70 Gew.-%, bevorzugt 20 bis 60 Gew.-%, besonders bevorzugt 25 bis 50 Gew.-%, beträgt.

8. Zusammensetzungen nach mindestens einem der Ansprüche 1 bis 7, **dadurch gekennzeichnet, dass** das Gewichtsverhältnis von verzweigten Alkoholen b1) zu unverzweigten Alkoholen b2) 90 : 10 bis 10 : 90, bevorzugt 70 : 30 bis 30 : 70, besonders bevorzugt 60 : 40 bis 40 : 60, beträgt.

9. Zusammensetzungen nach mindestens einem der Ansprüche 1 bis 8, **dadurch gekennzeichnet, dass** die verzweigten Alkohole b1) und die unverzweigten Alkohole b2) jeweils 10 bis 24, bevorzugt 12 bis 18, besonders bevorzugt 14 bis 16, Kohlenstoffatome besitzen.

10. Zusammensetzungen nach mindestens einem der Ansprüche 1 bis 9, **dadurch gekennzeichnet, dass** es sich bei den verzweigten Alkohole b1) um Guerbetalkohole, 2-Methyl-1-decanol, 2-Ethyl-1-nonanol, 2-Propyl-1-octanol, 2-Butyloctanol, 2-Butyl-decanol, 2-Hexyloctanol, 2-Hexyldecanol, 2-Octyldecanol, 2-Hexyldodecanol, 2-Octyldodecanol, 2-Decyltetradecanol, 2-Butyl-1-heptanol, 2-Dodecylhexadecanol, Tetradecyloctadecanol, 2-Tetradecyleicosanol, 2-Hexadecyloctadecanol, 2-Hexadecyleicosanol, 2-Methyltridecanol, 2-Ethyldodecanol, 2-Propylundecanol, 2-Pentylnonanol, 2-Heptylheptanol, 2-Methyltetradecanol, 2-Ethyltridecanol, 2-Propyldodecanol, 2-Butylundecanol, 2-Pentyldecanol, 2-Hexylnonanol, 2-Heptyloctanol, Isotridecylalkohol und/oder deren Mischungen handelt.

11. Zusammensetzungen nach mindestens einem der Ansprüche 1 bis 10, **dadurch gekennzeichnet, dass** es sich bei den unverzweigten Alkohole b2) um Fettalkohole, bevorzugt Lauryl-, Myristyl-, Cetyl-, Stearyl, Eicosanyl- und/oder Behenylalkohol, und/oder Nonanol, Undecanol, Tridecanol, Pentadecanol und/oder Heptadecanol handelt.

12. Zusammensetzungen nach mindestens einem der Ansprüche 1 bis 11, **dadurch gekennzeichnet, dass** sie, bezogen auf die fertigen Zusammensetzungen, weniger als 5 Gew.-%, bevorzugt weniger als 1 Gew.-%, an unverzweigten oder verzweigten Monoalkoholen mit 1 bis 4 Kohlenstoffatomen enthalten.

13. Zusammensetzungen nach mindestens einem der Ansprüchen 1 bis 12, **dadurch gekennzeichnet, dass** sie Stockpunkte unterhalb 100°C, bevorzugt unterhalb 95°C, besonders bevorzugt unterhalb 90°C, insbesondere bevorzugt unterhalb 85 °C, besitzen.

14. Zusammensetzungen nach mindestens einem der Ansprüche 1 bis 13, **dadurch gekennzeichnet, dass** sie Flammpunkte oberhalb 80°C, bevorzugt oberhalb 100°C, besitzen.

15. Verfahren zur Herstellung von Zusammensetzungen gemäß mindestens einem der Ansprüche 1 bis 14, **dadurch gekennzeichnet, dass** eine Mischung hergestellt wird, enthaltend
i) mindestens eine quaternäre Ammoniumverbindung a), gegebenenfalls enthaltend einen verzweigten oder unverzweigten Monoalkohol mit 1 bis 4 Kohlenstoffatomen,
ii) mindestens einen verzweigten Alkohol b1) mit 8 bis 36 Kohlenstoffatomen oder eine Mischung aus mindestens einem verzweigten Alkohol b1) und mindestens einem unverzweigten Alkohol b2) mit 8 bis 36 Kohlenstoffatomen und
iii) gegebenenfalls mindestens einen unverzweigten oder verzweigten Monoalkohol mit 1 bis 4 Kohlenstoffatomen.

16. Verfahren zur Herstellung einer Zusammensetzung gemäß mindestens einem der Ansprüche 1 bis 14, **dadurch gekennzeichnet, dass**
i) mindestens ein tertiäres Amin NR₁R₂R₃, wobei
R₁ für eine unverzweigte oder verzweigte Alkyl- oder Alkenylgruppe mit 12 bis 36 Kohlenstoffatomen, eine Gruppe R₅CONH(CH₂)ₙ- oder eine Gruppe R₅COO(CH₂)ₙ- steht, wobei R₅ für eine Alkyl- oder Alkenylgruppe mit 12 bis 36 Kohlenstoffatomen und n für eine Zahl von 1 bis 8 stehen,
und R₂ und R₃ unabhängig voneinander gleich oder verschieden sein können und für -CH₃, CH₃CH₂-, CH₃CH₂CH₂-, CH₃CH₂CH₂CH₂- oder -CH₂CH₂(OH) stehen,
durch
ii) mindestens ein Alkylierungsmittel, ausgewählt aus
a) R₄X, wobei R₄ für CH₃-, CH₃CH₂-, CH₃CH₂CH₂-, CH₃CH₂CH₂CH₂- oder -CH₂CH(OH)CH₂(OH) steht und X für Cl, I, Br, OSO₃H oder Methosulfat steht, und/oder
b) Ethylenoxid und einer Säure HX, wobei X für Cl, I, Br, OSO₃H, Citrat oder Lactat steht,
in Gegenwart von
iii) mindestens einem verzweigten Alkohol b1) mit 8 bis 36 Kohlenstoffatomen oder einer Mischung aus mindestens einem verzweigten Alkohol b1) und einem unverzweigten Alkohol b2) mit 8 bis 36 Kohlenstoffatomen und
iv) gegebenenfalls mindestens einem unverzweigten oder verzweigten Monoalkohol mit 1 bis 4 Kohlenstoffatomen
alkyliert wird.

17. Verwendung von Zusammensetzungen gemäß mindestens einem der Ansprüche 1 bis 14 zur Herstellung von kosmetischen, dermatologischen und pharmazeutischen Mitteln, bevorzugt Haarbehandlungsmitteln.

18. Verwendung nach Anspruch 17, **dadurch gekennzeichnet, dass** es sich bei den Haarbehandlungsmitteln um Shampoos, rinse-off-Haarconditioner, Cremespülungen, Klarspülungen, Haarkuren, Haarfärbe- und Haartönungsmittel, Dauerwellmittel, Haargele oder Haarconditioner in Aerosol-, Spray- und Fluidform handelt.

## Claims

1. A composition comprising, based on the finished composition,
a) 30% to 90% by weight of at least one quaternary ammonium compound according to formula (1) 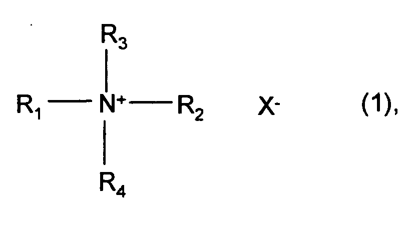 where
R₁ is an unbranched or branched alkyl or alkenyl group having 12 to 36 carbon atoms, a group R₅CONH(CH₂)ₙ- or a group R₅COO(CH₂)ₙ-, where R₅ is an alkyl or alkenyl group having 12 to 36 carbon atoms and n is a
number from 1 to 8,
and
R₂, R₃ and R₄, independently of one another, may be identical or different and are a -CH₃, CH₃CH₂-, CH₃CH₂CH₂-, CH₃CH₂CH₂CH₂-, CH₂CH₂OH or
CH₂CH(OH)CH₂OH group
and
X- is an anion,
and
b) at least one branched alcohol b1) having 8 to 36 carbon atoms or a mixture of at least one branched alcohol b1) and at least one unbranched alcohol b2) having 8 to 36 carbon atoms, which is free from polyhydric alcohols having 2 to 6 carbon atoms and is in the form of pellets or flakes.

2. The composition as claimed in claim 1, which comprises, as component b), a mixture of at least one branched alcohol b1) and at least one unbranched alcohol b2).

3. The composition as claimed in claim 1 and/or 2, wherein the content of quaternary ammonium compounds a), based on the finished composition, is 40 to 90% by weight, preferably 40 to 80% by weight, particularly preferably 45 to 75% by weight.

4. The composition as claimed in at least one of claims 1 to 3, wherein the quaternary ammonium compounds a) are (C₁₂-C₃₆)-, preferably (C₁₄₋C₃₀)-, particularly preferably (C₁₆-C₂₄)-alkyltrimethylammonium compounds.

5. The composition as claimed in at least one of claims 1 to 4, wherein the anion X⁻ in formula (1) is chloride, iodide, bromide, methosulfate, hydrogensulfate, lactate and/or citrate, preferably chloride and/or methosulfate.

6. The composition as claimed in at least one of claims 1 to 5, wherein the quaternary ammonium compound a) is behenyltrimethylammonium chloride.

7. The composition as claimed in at least one of claims 1 to 6, wherein the content of alcohols b), based on the finished composition, is 10 to 70% by weight, preferably 20 to 60% by weight, particularly preferably 25 to 50% by weight.

8. The composition as claimed in at least one of claims 1 to 7, wherein the weight ratio of branched alcohols b1) to unbranched alcohols b2) is 90:10 to 10:90, preferably 70:30 to 30:70, particularly preferably 60:40 to 40:60.

9. The composition as claimed in at least one of claims 1 to 8, wherein the branched alcohols b1) and the unbranched alcohols b2) each have 10 to 24, preferably 12 to 18, particularly preferably 14 to 16, carbon atoms.

10. The composition as claimed in at least one of claims 1 to 9, wherein the branched alcohols b1) are Guerbet alcohols, 2-methyl-1-decanol, 2-ethyl-1-nonanol, 2-propyl-1-octanol, 2-butyloctanol, 2-butyldecanol, 2-hexyloctanol, 2-hexyldecanol, 2-octyldecanol, 2-hexyldodecanol, 2-octyldodecanol, 2-decyltetradecanol, 2-butyl-1-heptanol, 2-dodecylhexadecanol, tetradecyloctadecanol, 2-tetradecyleicosanol, 2-hexadecyloctadecanol, 2-hexadecyleicosanol, 2-methyltridecanol, 2-ethyldodecanol, 2-propylundecanol, 2-pentyinonanol, 2-heptylheptanol, 2-methyltetradecanol, 2-ethyltridecanol, 2-propyldodecanol, 2-butylundecanol, 2-pentyldecanol, 2-hexylnonanol, 2-heptyloctanol, isotridecyl alcohol and/or mixtures thereof.

11. The composition as claimed in at least one of claims 1 to 10, wherein the unbranched alcohols b2) are fatty alcohols, preferably lauryl alcohol, myristyl alcohol, cetyl alcohol, stearyl alcohol, eicosanyl alcohol and/or behenyl alcohol, and/or nonanol, undecanol, tridecanol, pentadecanol and/or heptadecanol.

12. The composition as claimed in at least one of claims 1 to 11, which comprises, based on the finished composition, less than 5% by weight, preferably less than 1% by weight, of unbranched or branched monoalcohols having 1 to 4 carbon atoms.

13. The composition as claimed in at least one of claims 1 to 12, which has a setting point below 100°C, preferably below 95°C, particularly preferably below 90°C, especially preferably below 85°C.

14. The composition as claimed in at least one of claims 1 to 13, which has a flash point above 80°C, preferably above 100°C.

15. A process for the preparation of compositions as claimed in at least one of claims 1 to 14, which comprises preparing a mixture comprising
i) at least one quaternary ammonium compound a), optionally containing a branched or unbranched monoalcohol having 1 to 4 carbon atoms,
ii) at least one branched alcohol b1) having 8 to 36 carbon atoms or a mixture of at least one branched alcohol b1) and at least one unbranched alcohol b2) having 8 to 36 carbon atoms, and
iii) optionally at least one unbranched or branched monoalcohol having 1 to 4 carbon atoms.

16. A process for the preparation of a composition as claimed in at least one of claims 1 to 14, which comprises alkylating
i) at least one tertiary amine NR₁R₂R₃, where
R₁ is an unbranched or branched alkyl or alkenyl group having 12 to 36 carbon atoms, a group R₅CONH(CH₂)ₙ- or a group R₅COO(CH₂)ₙ-, where R₅ is an alkyl or alkenyl group having 12 to 36 carbon atoms and n is a number from 1 to 8,
and R₂ and R₃, independently of one another, may be identical or different and are -CH₃, CH₃CH₂-, CH₃CH₂CH₂-, CH₃CH₂CH₂CH₂- or -CH₂CH₂(OH),
by
ii) at least one alkylating agent chosen from
a) R₄X, where R₄ is -CH₃-, CH₃CH₂-, CH₃CH₂CH₂-, CH₃CH₂CH₂CH₂- or
-CH₂CH(OH)CH₂(OH), and X is Cl, I, Br, OSO₃H or methosulfate, and/or
b) ethylene oxide and an acid HX, where X is Cl, I, Br, OSO₃H, citrate or lactate,
in the presence of
iii) at least one branched alcohol b1) having 8 to 36 carbon atoms or a mixture of at least one branched alcohol b1) and one unbranched alcohol b2) having 8 to 36 carbon atoms and
iv) optionally at least one unbranched or branched monoalcohol having 1 to 4 carbon atoms.

17. The use of a composition as claimed in at least one of claims 1 to 14 for the preparation of cosmetic, dermatological and pharmaceutical compositions, preferably hair-treatment compositions.

18. The use as claimed in claim 17, wherein the hair-treatment compositions are shampoos, rinse-off hair conditioners, cream rinses, clear rinses, hair cures, hair colorants and hair tints, permanent waving compositions, hair gels and hair conditioners in aerosol form, spray form and fluid form.

## Revendications

1. Composition, contenant, par rapport à la composition prête à l'emploi,
a) 30 à 90 % en poids d'au moins un composé ammonium quaternaire selon la formule (1) 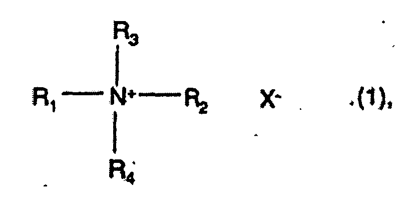 dans laquelle
R₁ représente un groupe alkyle ou alcényle non ramifié ou ramifié avec 12 à 36 atomes de carbone, un groupe R₅CONH(CH₂)ₙ- ou un groupe R₅COO(CH₂)ₙ-, dans lequel R₅ représente un groupe alkyle ou alcényle avec 12 à 36 atomes de carbone et n est un nombre de 1 à 8,
et
R₂, R₃ et R₄ indépendamment les uns des autres peuvent être identiques ou différents et représentent un groupe -CH₃, CH₃CH₂-, CH₃CH₂CH₂-, CH₃CH₂CH₂CH₂-, -CH₂CH₂OH
ou -CH₂CH(OH)CH₂OH
et
X⁻ représente un anion,
et
b) au moins un alcool ramifié b1) avec 8 à 36 atomes de carbone ou un mélange d'au moins un alcool ramifié b1) et d'au moins un alcool non ramifié b2) avec 8 à 36 atomes de carbone,
**caractérisé en ce qu'**elle est exempte d'alcool poly-hydroxylés avec 2 à 6 atomes de carbone et se présente sous la forme de pastilles ou de paillettes.

2. Composition selon la revendication 1, **caractérisée en ce qu'**elle contient comme composant b) un mélange d'au moins un alcool ramifié b1) et d'au moins un alcool non ramifié b2).

3. Composition selon la revendication 1 et/ou 2, **caractérisée en ce que** la teneur en composés ammonium quaternaire a), par rapport à la composition prête à l'emploi, s'élève à 40 à 90 % en poids, de préférence 40 à 80 % en poids, mieux encore 45 à 75 % en poids.

4. Composition selon au moins une des revendications 1 à 3, **caractérisée en ce qu'**il s'agit pour le composé ammonium quaternaire a) d'un composé (alkyle en C₁₂ à C₃₆)-, de préférence (alkyle en C₁₄ à C₃₀)-, mieux encore (alkyle en C₁₆ à C₂₄)-triméthyl-ammonium.

5. Composition selon au moins une des revendications 1 à 4, **caractérisée en ce qu'**il s'agit pour l'anion X⁻ dans la formule (1) de chlorure, iodure, bromure, méthosulfate, hydrogénosulfate, lactate et/ou citrate, de préférence chlorure et/ou méthosulfate.

6. Composition selon au moins une des revendications 1 à 5, **caractérisée en ce qu'**il s'agit pour le composé ammonium quaternaire a) du chlorure de béhényltriméthylammonium.

7. Composition selon au moins une des revendications 1 à 6, **caractérisée en ce que** la teneur en alcools b), par rapport à la composition prête à l'emploi, s'élève à 10 à 70 % en poids, de préférence 20 à 60% en poids, mieux encore 25 à 50 % en poids.

8. Composition selon au moins une des revendications 1 à 7, **caractérisée en ce que** le rapport en poids des alcools ramifiés b1) par rapport aux alcools non ramifiés b2) s'élève de 90 : 10 à 10 : 90, de préférence 70 : 30 à 30 : 70, mieux encore 60 : 40 à 40 : 60.

9. Composition selon au moins une des revendications 1 à 8, **caractérisée en ce que** les alcools ramifiés b1) et les alcools non ramifiés b2) possèdent respectivement 10 à 24, de préférence 12 à 18, mieux encore 14 à 16 atomes de carbone.

10. Composition selon au moins une des revendications 1 à 9, **caractérisée en ce qu'**il s'agit pour les alcools ramifiés b1) d'alcools de Guerbet, 2-méthyl-1-décanol, 2-éthyl-1-nonanol, 2-propyl-1-octanol, 2-butyloctanol, 2-butyldécanol, 2-hexyloctanol, 2-hexyldécanol, 2-octyldécanol, 2-hexyldodécanol, 2-octyldodécanol, 2-décyltétradécanol, 2-butyl-1-heptanol, 2-dodécylhexadécanol, tétradécyloctadécanol, 2-tétradécyleicosanol, 2-hexadécyloctadécanol, 2-hexadécyleicosanol, 2-méthyltridécanol, 2-éthyldodécanol, 2-propylundécanol, 2-pentylnonanol, 2-heptylheptanol, 2-méthyltétradécanol, 2-éthyltridécanol, 2-propyldodécanol, 2-butylundécanol, 2-pentyldécanol, 2-hexylnonanol, 2-heptyloctanol, isotridécylalcool et/ou leurs mélanges.

11. Composition selon au moins une des revendications 1 à 10, **caractérisée en ce qu'**il s'agit pour les alcools non ramifiés b2) d'alcools gras, de préférence le lauryl-, myristyl-, cétyl-, stéaryl-, eicosanyl- et/ou béhénylalcool, et/ou le nonanol, undécanol, tridécanol, pentadécanol et/ou heptadécanol.

12. Composition selon au moins une des revendications 1 à 11, **caractérisée en ce qu'**elle contient, par rapport à la composition prête à l'emploi, moins de 5 % en poids, de préférence moins de 1 % en poids, de mono-alcools non ramifiés ou ramifiés avec 1 à 4 atomes de carbone.

13. Composition selon au moins une des revendications 1 à 12, **caractérisée en ce qu'**elle possède un point d'écoulement inférieur à 100 °C, de préférence inférieur à 95 °C, mieux encore inférieur à 90 °C, en particulier inférieur à 85 °C.

14. Composition selon au moins une des revendications 1 à 13, **caractérisée en ce qu'**elle possède un point d'inflammation au-dessus de 80 °C, de préférence au-dessus de 100 °C.

15. Procédé de préparation de compositions selon au moins l'une des revendications 1 à 14, **caractérisé en ce qu'**on prépare un mélange, contenant
i) au moins un composé ammonium quaternaire a), contenant éventuellement un mono-alcool ramifié ou non ramifié avec 1 à 4 atomes de carbone,
ii) au moins un alcool ramifié b1) avec 8 à 36 atomes de carbone ou un mélange d'au moins un alcool ramifié b1) et d'au moins un alcool non ramifié b2) avec 8 à 36 atomes de carbone et
iii) éventuellement au moins un mono-alcool non ramifié ou ramifié avec 1 à 4 atomes de carbone.

16. Procédé de préparation d'une composition selon au moins l'une des revendications 1 à 14, **caractérisé en ce qu'**on alkyle
i) au moins une amine tertiaire NR₁R₂R₃, dans laquelle
R₁ représente un groupe alkyle ou alcényle ramifié ou non ramifié avec 12 à 36 atomes de carbone, un groupe R₅CONH(CH₂)ₙ- ou un groupe R₅COO(CH₂)ₙ-, dans lequel R₅ représente un groupe alkyle ou alcényle avec 12 à 36 atomes de carbone et n est un nombre de 1 à 8,
et R₂ et R₃ indépendamment l'un de l'autre peuvent être identiques ou différents et représentent -CH₃, CH₃CH₂-, CH₃CH₂CH₂-, CH₃CH₂CH₂CH₂- ou -CH₂CH₂(OH),
au moyen de
ii) au moins un agent alkylant, choisi parmi
a) R₄X, dans lequel R₄ représente -CH₃, CH₃CH₂-, CH₃CH₂CH₂-, CH₃CH₂CH₂CH₂- ou -CH₂CH(OH)CH₂(OH) et X représente Cl, I, Br, OSO₃H ou un méthosulfate, et/ou
b) l'oxyde d'éthylène et un acide HX, dans lequel X représente Cl, I, Br, OSO₃H, un citrate ou lactate,
en présence de
iii) au moins un alcool ramifié b1) avec 8 à 36 atomes de carbone ou un mélange d'au moins un alcool ramifié b1) et un alcool non ramifié b2) avec 8 à 36 atomes de carbone et
iv) éventuellement au moins un mono-alcool non ramifié ou ramifié avec 1 à 4 atomes de carbone.

17. Utilisation de compositions selon au moins l'une des revendications 1 à 14 pour la préparation d'agents cosmétiques, dermatologiques et pharmaceutiques, de préférence de préparations capillaires.

18. Utilisation selon la revendication 17, **caractérisée en ce qu'**il s'agit pour les préparations capillaires de shampooings, revitalisants capillaires sans rinçage, après-shampooings, agents mouillants, produits de soins capillaires, colorants capillaires et teintures capillaires, compositions de permanentes, gels capillaires et conditionneurs capillaires sous forme d'aérosol, de pulvérisation ou de liquide.
